**Europäisches Patentamt**

**European Patent Office**

**Office européen des brevets**

(19)

(11) Publication number : **0 436 726 B1**

(12) # EUROPEAN PATENT SPECIFICATION

(45) Date of publication of patent specification :
20.10.93 Bulletin 93/42

(51) Int. Cl.⁵ : **A61K 9/08, A61K 47/02,
A61K 47/10**

(21) Application number : **90910883.9**

(22) Date of filing : **26.07.90**

(86) International application number :
**PCT/JP90/00950**

(87) International publication number :
**WO 91/01718 21.02.91 Gazette 91/05**

(54) **METHOD OF PHOTOSTABILIZING EYEWASH.**

(30) Priority : **03.08.89 JP 201872/89**

(43) Date of publication of application :
**17.07.91 Bulletin 91/29**

(45) Publication of the grant of the patent :
**20.10.93 Bulletin 93/42**

(84) Designated Contracting States :
**AT BE CH DE DK ES FR GB IT LI LU NL SE**

(56) References cited :
**WO-A-85/04106
GB-A- 2 199 745
US-A- 2 445 366
US-A- 3 966 905**

(73) Proprietor : **Eisai Co., Ltd.
6-10, Koishikawa 4-chome Bunkyo-ku
Tokyo 112 (JP)**

Proprietor : **SANTEN PHARMACEUTICAL CO.,
LTD.
9-19, Shimoshinjo 3-chome
Higashiyodogawa-ku Osaka-shi Osaka 533
(JP)**

(72) Inventor : **MORITA, Takakazu 302
Kitasakurazuka Park Heim
6-8, Kitasakurazuka 3-chome Toyonaka-shi
Osaka 560 (JP)**
Inventor : **MITA, Shiro
26-304, Higashiyamacho 7-chome Ashiya-shi
Hyogo 659 (JP)**
Inventor : **KAWASHIMA, Yoichi
8-54, Oharanonishisakaidanicho 3-chome
Nishikyo-ku Kyoto-shi Kyoto 610-11 (JP)**

(74) Representative : **Pearce, Anthony Richmond et
al
MARKS & CLERK Alpha Tower Suffolk Street
Queensway Birmingham B1 1TT (GB)**

EP 0 436 726 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the photostabilizing of ophthalmic solutions which contain drug substances which are unstable to light.

There are many drugs which are unstable against light and therefore such drugs are put in practical use by shielding light or by a special pharmaceutical design to prevent decomposition during use.

An aqueous preparation of a drug substance is more easily decomposed by light than a solid form, and such decomposition may cause coloring of the aqueous preparation, etc.

In case of an aqueous preparation such as an ophthalmic solution, it has so far been difficult to solve the problem of decomposition by light by the pharmaceutical design. Therefore, decomposition has been prevented by shielding light.

However, complete blocking of light is practically difficult, and thus we have reverted to improvement of the formulation to stabilize the solution itself.

Ingredients which can be formulated in ophthalmic solutions are restricted because ophthalmic solutions are administered to the eyes which are highly sensitive organs. Such restriction causes one of the severe obstacles to the design of photostable ophthalmic solutions.

US-A-3966905 discloses an ophthalmic solution containing a catechol amine, such as epinephrine, which is unstable to light. Such solution is stabilized by use of a borate which forms a complex with the catechol amine and which acts as a buffer, a polyvinylpyrrolidone polymer, and a physiologically acceptable antioxidant.

The present invention resides in the use of a mixture of a polyhydric alcohol and boric acid, and/or sodium borate, for photostabilizing an ophthalmic solution which contains a drug which is unstable to light.

There are various drug substances which are unstable against light, and it is considered that substances which have an aromatic ring substituted by hydroxy, lower alkoxy, primary or secondary amine are generally unstable against light. Examples of such substances are bunazosin, prazosin, terazosin, epinephrine and phenylephrine. Of course, pharmacologically acceptable salts such as hydrochloric acid salt of drug substance can be used in this invention.

A polyhydric alcohol is a compound having a plurality of hydroxy groups and is not necessarily restricted to a specific compound. Examples of polyhydric alcohol are glycerol, polyethylene glycol, propylene glycol, mannitol and glucose.

Ophthalmic solutions which contain drug substance which are unstable to light can be put in practical use by shielding from the light. However, it is practically difficult to shield light completely, and thus it is desired to stabilize the solution itself by an improvement of the formulation. Ingredients have to be taken into special consideration because ophthalmic solutions are administered to the eyes which are highly sensitive organs. As the result of our precise study on a photostabilizing method for ophthalmic solutions, we found that photostability of ophthalmic solutions could be attained by adding polyhydric alcohol and boric acid, and/or sodium borate, which have been confirmed as safe additives to ophthalmic solutions. The detailed photostabilizing mechanism of polyhydric alcohol and boric acid, and/or sodium borate is not clear, but it is presumed that the photostability might be attained by conforming a complex with boron and polyhydric alcohol in the aqueous solution. Accordingly, this invention can be widely applied to any drug substances which can conform such complex, and the application should not be restricted to the above-mentioned drug substances. In case of bunazosin hydrochloride, which has a suppressive effect on intraocular pressure, the ophthalmic solutions adding polyhydric alcohol and boric acid, and/or sodium borate were not colored after exposure to an intensive light such as 3000 luxes for 200 hours. On the other hand, the ophthalmic solutions adding only polyhydric alcohol or boric acid were decomposed and colored at the same condition. The details are explained in an article of the stability test. As the result of our study, we found that the photostability of the ophthalmic solutions can be attained by a combination of polyhydric alcohol and boric acid, and/or sodium borate, while an addition of boric acid or polyhydric alcohol only is not effective on the photostability.

In this invention, the optimal amount of boric acid or sodium borate is changeable. The amount can be decided according to a nature of drug substance and the concentration thereof, but the preferable amount is 0.5 - 2.5%.

The optimal amount of polyhydric alcohol is changeable. The amount can be decided according to a nature of drug substance and the concentration thereof, but the preferable amount of is 0.1 - 2.0%.

The ophthalmic solutions making use of this invention can be prepared by the known methods. The outline of the preparative method is that polyhydric alcohol and boric acid, and/or sodium borate are added and dissolved in an aqueous solution of the drug substance which is unstable to light, and if necessary tonicity agents such as sodium chloride and potassium chloride, stabilizer such as disodium edetate, preservatives such as benzalkonium chloride and pH adjusting agents such as sodium hydroxide and dilute hydrochloric acid can be formulated.

2

The pH value of the ophthalmic solutions making use of this invention can be adjusted within a range applicable to medicine and should not be restricted, but the preferable range is 4.5 - 8.

Examples are given below.

EXAMPLES

Formulation 1

| | |
|---|---|
| bunazosin hydrochloride | 0.1g |
| boric acid | 1.24g |
| conc. glycerol | 0.3g |
| benzalkonium chloride | 0.005g |
| sodium hydroxide | q.s. |
| sterile purified water | q.s. |
| total | 100ml |

Preparation Method :

Bunazosin hydrochloride, conc. glycerol and benzalkonium chloride were dissolved in sterile purified water and the pH of the solution was adjusted to 6.0. To the solution, sterile purified water was added to adjust the total volume to 100ml.

The following ophthalmic solutions were prepared by a similar method to the above.

Formulation 2

| | |
|---|---|
| bunazosin hydrochloride | 0.1g |
| boric acid | 1.0g |
| conc. glycerol | 0.5g |
| benzalkonium chloride | 0.005g |
| sodium chloride | 0.23g |
| sodium hydroxide | q.s. |
| sterile purified water | q.s. |
| total | 100ml ( pH6.0 ) |

Formulation 3

| | |
|---|---|
| bunazosin hydrochloride | 0.01g |
| boric acid | 1.24g |
| mannitol | 0.6g |
| benzalkonium chloride | 0.005g |
| sodium hydroxide | q.s. |
| sterile purified water | q.s. |
| total | 100ml ( pH6.0 ) |

Formulation 4

| | |
|---|---|
| bunazosin hydrochloride | 0.1g |
| boric acid | 1.4g |
| mannitol | 0.5g |
| benzalkonium chloride | 0.005g |
| sodium hydroxide | q.s. |
| sterile purified water | q.s. |
| total | 100ml ( pH6.0 ) |

Formulation 5

| | |
|---|---|
| epinephrine bitartrate | 2.0g |
| sodium borate | 2.5g |
| polyethylene glycol | 0.9g |
| benzalkonium chloride | 0.005g |
| sodium edetate | 0.01g |
| dilute hydrochloric acid | q.s. |
| sterile purified water | q.s. |
| total | 100ml ( pH7.5 ) |

Formulation 6

| | |
|---|---|
| prazosin hydrochloride | 0.05g |
| boric acid | 1.0g |
| conc. glycerol | 0.5g |
| benzalkonium chloride | 0.005g |
| sodium hydroxide | q.s. |
| sterile purified water | q.s. |
| total | 100ml ( pH6.0 ) |

Formulation 7

| | |
|---|---|
| bunazosin hydrochloride | 0.01g |
| boric acid | 2.0g |
| propylene glycol | 0.1g |
| benzalkonium chloride | 0.005g |
| sodium edetate | 0.01g |
| sodium hydroxide | q.s. |
| sterile purified water | q.s. |
| total | 100ml ( pH6.0 ) |

Formulation 8

| | |
|---|---|
| bunazosin hydrochloride | 0.5g |
| boric acid | 0.5g |
| conc. glycerol | 2.0g |
| sodium hydroxide | q.s. |
| sterile purified water | q.s. |
| total | 100ml ( pH5.5 ) |

Formulation 9

| | |
|---|---|
| bunazosin hydrochloride | 0.1g |
| boric acid | 1.24g |
| sodium borate | 0.1g |
| conc. glycerol | 0.3g |
| benzalkonium chloride | 0.005g |
| sodium hydroxide | q.s. |
| dilute hydrochloric acid | q.s. |
| sterile purified water | q.s. |
| total | 100ml ( pH6.0 ) |

STABILITY TEST

The photostability of ophthalmic solutions containing bunazosin hydrochloride as a typical drug substance was examined.

Experimental Method :

The ophthalmic solution of the formulation 1 put in polypropylene eye dropper bottle was exposed to light at 3000 lux for 200 hours and the change of color was examined.

By way of comparison, the following two ophthalmic solutions were prepared and compared with the ophthalmic solution of the formulation 1.

Reference 1

| | |
|---|---|
| bunazosin hydrochloride | 0.1g |
| boric acid | 1.24g |
| benzalkonium chloride | 0.005g |
| sodium hydroxide | q.s. |
| sterile purified water | q.s. |
| total | 100ml |

Reference 2

| | |
|---|---|
| bunazosin hydrochloride | 0.1g |
| conc. glycerol | 0.3g |
| benzalkonium chloride | 0.005g |
| sodium hydroxide | q.s. |
| sterile purified water | q.s. |
| total | 100ml |

Experimental Result :

Table 1 shows the observation result before and after the exposure to light.

Table 1

| | before exposure | after exposure |
|---|---|---|
| Formulation 1 | colorless | colorless |
| Reference 1 | colorless | light red |
| Reference 2 | colorless | light red |

As shown in Table 1, the colors of the ophthalmic solutions adding boric acid ( Reference 1 ) and conc. glycerol ( Reference 2 ) were changed from colorless to light red as a result of decomposition by light. On the other hand, coloration was not observed in the ophthalmic solution of the Formulation 1 containing a mixture of boric acid and conc. glycerol, and prevention of decomposition by light was achieved. When bunazosin hydrochloride was excluded from formulation 1, no influence of light was observed.

As the result of the experiment, we found that the photo-stability of the ophthalmic solutions could be attained by combining polyhydric alcohol and boric acid, and/or sodium borate, while the addition of boric acid or polyhydric alcohol without the combination each other was not effective on photostability.

By this invention, unstable drug substance against light can be easily applicable to ophthalmic solutions by combining polyhydric alcohol and boric acid, and/or sodium borate.

**Claims**

1. The use of a mixture of a polyhydric alcohol and boric acid, and/or sodium borate, for photostabilising an ophthalmic solution which contains a drug which is unstable to light.

2. The use as claimed in claim 1, wherein the drug is a compound which has aromatic ring substituted by hydroxy, lower alkoxy, primary or secondary amine, or a salt thereof.

3. The use as claimed in claim 1, wherein the drug is bunazosin hydrochloride.

4. The use as claimed in claim 1, wherein the drug is bunazosin hydrochloride and the polyhydric alcohol is glycerol.

**Patentansprüche**

1. Verwendung eines Gemisches eines Polyhydroxyalkohols und Borsäure und/oder Natriumborats zur Photostabilisierung einer ophthalmischen Lösung, die einen Wirkstoff enthält, der gegenüber Licht nicht stabil ist.

2. Verwendung nach Anspruch 1, dadurch gekennzeichnet, daß der Wirkstoff eine Verbindung, die einen aromatischen Ring aufweist, der durch eine Hydroxy-, Niederalkoxy-, primäre oder sekundäre Amingruppe substituiert ist, oder deren Salz ist.

3. Verwendung nach Anspruch 1, dadurch gekennzeichnet, daß der Wirkstoff Bunazosinhydrochlorid ist.

4. Verwendung nach Anspruch 1, dadurch gekennzeichnet, daß der Wirkstoff Bunazosinhydrochlorid und der Polyhydroxyalkohol Glycerin ist.

**Revendications**

1. Utilisation d'un mélange d'un polyol et d'acide borique et/ou de borate de sodium pour photostabiliser une solution ophtalmique qui contient un médicament qui est instable à la lumière.

2. Utilisation telle que revendiquée dans la revendication 1, dans laquelle le médicament est un composé qui a un noyau aromatique substitué par un hydroxy, un alcoxy inférieur, une amine primaire ou secondaire ou un de ses sels.

3. Utilisation telle que revendiquée dans la revendication 1, dans laquelle le médicament est le chlorhydrate de bunazosine.

4. Utilisation telle que revendiquée dans la revendication 1, dans laquelle le médicament est le chlorhydrate de bunazosine et le polyol est le glycérol.